# EUROPÄISCHE PATENTANMELDUNG

(11) **EP 0 847 729 A1**
(43) Veröffentlichungstag der Anmeldung: **17.06.1998**
(21) Anmeldenummer: 96810866.2
(22) Anmeldetag: 12.12.1996
(51) Int. Cl.: A61B 17/39

(54) **Ablationsgerät für intrakardiale Herzbehandlungen**

(71) Anmelder: Sulzer Osypka GmbH, 79639 Grenzach-Wyhlen (DE)
(72) Erfinder: Geistert, Wolfgang, Dr., 79618 Rheinfelden-Herten (DE); Bothur, Markus, 79599 Wittlingen (DE); Römer, Ferdinand, 79872 Bernau (DE)
(74) Vertreter: Heubeck, Bernhard

(57) **Zusammenfassung**

Das Ablationsgerät für intrakardiale Herzbehandlungen weist eine Hochfrequenz-Energiequelle auf, für deren Leistungsabgabe eine Temperaturregelung vorgesehen ist. Es weist ferner mindestens einem Ablationskatheter auf, der mindestens einen energieabgebenden Pol umfasst, wobei der oder jeder Pol mindestens einen Temperatursensor zur Messung einer Gewebetemperatur, nämlich eines Temperatur-Istwerts, enthält. Mit dieser Messung ist jeweils die Soll/Ist-Differenz zwischen dem Temperatur-Istwert und einer vorgegebenen Solltemperatur bestimmbar. Es können für die Beobachtung des bei der Ablation entstehenden thermischen Einflusses mehr als ein Temperatursensor vorgesehen sein. Die Regelung umfasst Mittel, aufgrund derer die Leistungsabgabe so steuerbar ist, dass die grösste der durch die verschiedenen Temperatursensoren gemessenen Temperaturen einen vorgegebenen Grenzwert nicht wesentlich überschreitet. Mit Vorteil ist die Leistungsabgabe mittels einer "Fuzzy-Logik"-Regelung einstellbar, wobei mindestens eine der genannten Soll/Ist-Temperaturdifferenzen als Eingangsvariable der Regelung verwendet wird.

## Beschreibung

Die Erfindung betrifft ein Ablationsgerät für intrakardiale Herzbehandlungen mit einer Hochfrequenz-Energiequelle (kurz HF-Quelle) gemäss Oberbegriff von Anspruch 1.

Bei der Ablation werden im Herz mittels eines flexiblen Katheters Gewebeschichten denaturiert, um Störungen des Erregungsleitsystems zu beseitigen. Solche Störungen äussern sich in Herzarhythmien (z. B. Tachykardie). Die störenden Erregungsleitungen werden mit dem Ablationskatheter lokalisiert und anschliessend gezielt unterbrochen. Die für die Unterbrechung vorgenommene Gewebeläsion wird mit Hilfe hochfrequenter Wechselströme einer HF-Energiequelle durchgeführt. Die Leistungsabgabe der HF-Quelle wird mit einer Temperaturregelung so eingestellt, dass die Zellen abgetötet werden, aber der Zellverband (das Gewebe) nicht zerstört wird, und dass an dem energieabgebenden Pol keine Koagulation auftritt.

Bei bekannten Ablationsgeräten mit Temperaturregelung wird diese bei einfacheren Ausführungsformen mit P-Reglern und bei aufwändigeren beispielsweise mit PID-Regler durchgeführt. Der energieabgebende Pol enthält einen Temperatursensor zur Messung der Gewebetemperatur, die den Temperatur-Istwert darstellt. Die Soll/Ist-Differenz zwischen dem Temperatur-Istwert und einer vorgegebenen Solltemperatur ist beim P-Regler die einzige Eingangsvariable. Beim PID-Regler wird zusätzlich zu dem durch die Soll/Ist-Temperaturdifferenz gegebenen P-Anteil auch ein I- sowie ein D-Anteil berücksichtigt. Der D-Anteil ist die Ableitung des Temperatur-Istwerts nach der Zeit, d. h. die Geschwindigkeit der Temperaturänderung. Der I-Anteil ist das Integral der Soll/Ist-Temperaturdifferenz über die Zeit und stellt damit ein Mass für den Temperaturverlauf bis zum momentanen Zeitpunkt dar.

Bei der Durchführung der Ablation sind zwei Punkte wichtig: 1. Die Gewebetemperatur soll möglichst rasch den für die Gewebeläsion nötigen Wert erreichen. 2. Die Gewebetemperatur soll aber möglichst wenig über den angezielten Wert hinausschiessen, damit eine Überhitzung und eine mit dieser verbundenen Koagulation des Gewebes ausbleibt.

Für die vorliegende Erfindung ist folgende Erkenntnis grundlegend: Im schlagenden Herz kann der Pol des Ablationskatheters ständig etwas hin- und hergleiten. Der Pol bewegt sich dabei zwischen Stellen, die unterschiedliche Wärmeableitungen aufweisen; er wird ausserdem durch wechselnde Blutströmungen gekühlt. Es liegen somit am Pol ständig wechselnde Verhältnisse vor. Folglich muss zur Aufrechterhaltung einer vorgegebenen Temperatur für eine wechselnde Leistungszufuhr gesorgt werden.

Aufgrund der Inhomogenitäten der Wärmeableitung kann auf dem Pol eine Temperaturverteilung mit unterschiedlichen Temperaturen entstehen. Der Temperatursensor misst eine mittlere Temperatur und somit können sich auf dem Pol lokale Überhitzungen einstellen.

Da wegen des schlagenden Herzes der Pol in der Regel nicht in einer stationären Lage bezüglich des zu behandelnden Gewebes bleibt, ist - bei einer konstanten Leistungsabgabe - die am Pol gemessene Temperatur aufgrund der Inhomogenitäten der Wärmeableitung grossen Schwankungen unterworfen. Wird zur Temperaturregelung ein P-Regler verwendet, so muss dieser sehr schnell ausgelegt sein, damit die Gewebetemperatur ausreichend stabil gehalten werden kann. Mit einem derartigen P-Regler sind aber stark fluktuierende Änderungen der Leistungsabgabe verbunden; und diese führen leicht zu Störungen der EKG-Messung, die während der Ablation zur Überwachung der Herztätigkeit vorgenommen werden müssen.

Die Verwendung eines PID-Reglers anstelle eines P-Reglers macht es möglich, schnelle Änderungen der Leistungsabgabe zu unterdrücken. Wegen den schnell ändernden Verhältnissen am Pol neigen die PID-Regler zum Schwingen. Um insbesondere ein Überschwingen klein zu halten, muss der PID-Regler relativ langsam eingestellt werden. Mit einer solchen Einstellung ergibt sich nun aber, dass die mittlere Gewebetemperatur nur langsam auf den benötigten Sollwert erhöht werden kann. Dies bedeutet für die Durchführung der Herzoperation einen Nachteil.

Diese Probleme, die mit Inhomogenitäten bei der Wärmeableitung des Herzgewebes zusammenhängen, sind bis jetzt noch nicht erkannt worden. Es ist auch nicht naheliegend gewesen, diese Probleme zu erkennen. Aufgabe der Erfindung ist es, Ablationsgeräte zu schaffen, bei denen die genannten Probleme in einem gegenüber den bekannten Geräten abgeschwächten Ausmass auftreten.

Diese Aufgabe wird durch die in den Ansprüchen - insbesondere in den unabhängigen Ansprüchen 1, 4 und 7 - definierten Geräte gelöst. Die abhängigen Ansprüche stellen vorteilhafte Ausführungsformen dar.

Nach der Lehre gemäss Anspruch 1 sind für die Beobachtung des bei der Ablation entstehenden thermischen Einflusses auf das Gewebe mehr als ein Temperatursensor vorgesehen. Die Regelung umfasst Mittel, aufgrund derer die Leistungsabgabe so steuerbar ist, dass die grösste der durch die verschiedenen Temperatursensoren gemessene Temperatur einen vorgegebenen Grenzwert nicht wesentlich, d.h. um mehr als rund 3-6°C, überschreitet. Der Grenzwert ist so gewählt, dass keine Koagulation entsteht.

Der Pol kann aus mehreren getrennten Segmenten zusammengesetzt sein, die alle an die gleiche HF-Quelle angeschlossen sind und die jeweils einen separaten Temperatursensor enthalten. Die Ablation kann auch mit zwei getrennten aber an der gleichen HF-Quelle angeschlossenen Kathetern, die auf die gleiche Gewebestelle einwirken, durchgeführt werden. In diesem Fall stellen die Pole der beiden Katheter zwei Segmente eines gemeinsamen Pols oder auch zwei Gegenpole dar.

Nach der Lehre gemäss Anspruch 4 ist für die Leistungsabgabe eine "Fuzzy-Logik"-Regelung vorgesehen. Dabei wird mindestens eine der genannten Soll/Ist-Temperaturdifferenzen als Eingangsvariable der Regelung verwendet. Dank dieser Art Regelung erscheinen nun nicht mehr die beim P-Regler festgestellten starken Fluktuationen der Leistungsabgabe. Die EKG-Diagnose wird somit weniger oder praktisch nicht mehr beeinträchtigt. Die Durchführung der "Fuzzy-Logik"-Regelung wird weiter unten anhand einer graphischen Darstellung erläutert.

Besonders vorteilhaft ist eine Kombination der beiden genannten Lehren (Anspruch 7): Verwendung eines energieabgebenden Pols mit mindestens zwei Temperatursensoren und einer "Fuzzy-Logik"-Regelung, die Mittel umfasst, aufgrund derer die HF-Leistungsabgabe gemäss Anspruch 1 steuerbar ist.

Liegt eine Mehrzahl von Temperatursensoren pro Pol vor, so kann vorgesehen sein, dass die Regelung der HF-Leistungsabgabe nur hinsichtlich der grössten gemessenen Temperatur vorgenommen wird. Es kann auch eine Differenz zwischen den durch die verschiedenen Temperatursensoren gemessenen Temperaturen als zusätzlicher Steuerwert für die HF-Leistungsabgabe verwendet werden. Auch eine Mehrzahl von solchen Temperaturdifferenzen können als Steuerwerte vorgesehen sein.

Ablationsgeräte setzen sich aus einem Geräteteil, das die HF-Quelle enthält, und mindestens einem an die HF-Quelle angeschlossenen Katheter zusammen. Es werden verschiedene Kathetertypen verwendet, beispielsweise solche, die sich durch die Temperatursensoren der Pole unterscheiden. Diese Sensoren können Thermistoren oder Thermoelemente sein. Mit Vorteil weist das Ablationsgerät eine Sensortyp-Erkennung auf sowie entsprechende Mittel, aufgrund derer bei der Regelung für die Leistungsabgabe automatisch die Messwerte des aktuellen Sensortyps verwendet werden.

Bei bekannten Ablationsgeräten muss der Kathetertyp per Hand eingestellt werden. Nebst dieser zusätzlichen Bedienhandlung besteht eine Verwechslungsgefahr. Durch die genannte Sensortyp-Erkennung wird die Umschaltung automatisch und zuverlässig vorgenommen.

Für die automatische Erkennung sind die Messschaltungen für den Thermistor bzw. das Thermoelement folgendermassen gestaltet: 1. Die Thermistor-Messschaltung kann die Zustände "offen", "Kurzschluss" und "gültiger Sensor" unterscheiden. 2. Die Thermoelement-Messschaltung kann die Zustände "offen" und "nicht offen" sowie mit Vorteil "sinnvoller Temperaturwert" unterscheiden. (Der Zustand "Kurzschluss" kann von der Thermoelement-Messschaltung nicht erkannt werden, da ein Thermoelement, das die gleiche Temperatur wie eine zugehörige Vergleichsstelle hat, als Kurzschluss erscheint.)

Der Erkennungsalgorithmus arbeitet wie folgt: 1. Wird durch die gerade aktive Messschaltung der Zustand "offen" erkannt, wird immer die Thermistor-Messschaltung eingeschaltet. 2. Erkennt die Thermistor-Messschaltung einen "gültigen Sensor", so lautet das Ergebnis der automatische Erkennung "Thermistor". 3. Erkennt die Thermistor-Messschaltung einen Kurzschluss, lautet das Ergebnis der automatische Erkennung "Thermoelement". (Die Thermistor-Messschaltung kann darauf deaktiviert und die Thermoelement-Messschaltung aktiviert werden.)

Zusätzlich zur Sensortyp-Erkennung sind mit Vorteil Mittel vorgesehen, mit denen ein Test oder mehrer Tests durchführbar sind, welche darauf gerichtet sind, ob angeschlossene Sensoren sinnvolle Temperaturwerte liefern: Die Temperaturwerte sollen in zu erwartenden Bereichen liegen. Bei Verwendung von Kathetern mit Thermoelementen kann mit Tests gegebenenfalls noch festgestellt werden, ob auch Vergleichsstellen der Thermoelemente sinnvolle Temperaturwerte liefern.

Vorteilhaft sind zusätzliche Mittel, mit denen die Messschaltung für die Temperaturmessung kalibrierbar und testbar sind. Ferner sind Mittel vorteilhaft, mit denen eine Überwachung einer Startphase durchführbar ist. Durch eine solche Überwachung soll verhindert werden, dass das Ablationsgerät in einem leistungsgeregelten Modus statt in dem temperaturgeregelten startet. Es handelt sich dabei um einen "intelligenten Start" des erfindungsgemässen Geräts.

In Bezug auf den "intelligenten Start" lassen sich noch folgende Ergänzungen anmerken:

Bekannt sind Ablationsgeräte, bei denen per Hand zwischen temperaturgeregeltem und leistungsgeregeltem Betrieb umgeschaltet werden muss. Es ist auch ein Gerät bekannt, bei dem diese Umschaltung automatisch geschieht. Dieses Gerät schaltet immer dann automatisch in den temperaturgeregeltem Betrieb um, wenn ein Katheter mit Temperatursensor erkannt wird. Liegt kein Katheter dieser Art vor, so schaltet das Gerät zurück in den leistungsgeregelten Betrieb. Das Gerät würde ebenfalls in diesem Betrieb starten, wenn ein defekter oder fehlerhafter Temperatursensor vorliegt oder das Anschlusskabel einen Wackelkontakt aufweist. Ein solcher versehentlicher Start muss aber vermieden werden. Dies leistet folgender Algorithmus, welcher der genannten Überwachung zugeordnet ist.

Sobald ein Katheter mit Temperatursensor angeschlossen wird und dies erkannt wird, aktiviert sich automatisch die Überwachung. Wird jetzt eine HF-Abgabe bei angeschlossenem Temperatursensor gestartet, so geht dies ohne weitere Bedienhandlung vonstatten, d. h. es wird eine HF-Abgabe im temperaturgeregelten Modus gestartet. Wurde jedoch mittlerweile ein Katheter ohne Temperatursensor angeschlossen oder ist der Temperaturmesskreis durch einen defekten Sensor oder Wackelkontakt geöffnet, so erfolgt eine Nachfrage, ob die HF-Abgabe wirklich im leistungsgeregelten Modus erfolgen soll. Wird dies verneint, wird der Vorgang abgebrochen (d. h. die HF-Abgabe nicht gestartet), und die Überwachung bleibt aktiv. Wird dies jedoch bejaht, wird die Überwachung deaktiviert. Die HF-Abgabe wird jetzt sofort im leistungsgeregelten Modus gestartet, oder sie kann mittels eines weiteren Tastendrucks gestartet werden. Von nun an können so lange weitere HF-Abgaben im leistungsgeregelten Modus gestartet werden, ohne dass der Start jedesmal bestätigt werden muss, bis wieder ein Katheter mit Temperatursensor angeschlossen wird. Mit Vorteil ist die Überwachung nach Einschalten des Geräts aktiviert, so dass zu Beginn immer ein Katheter mit Temperatursensor gefordert wird.

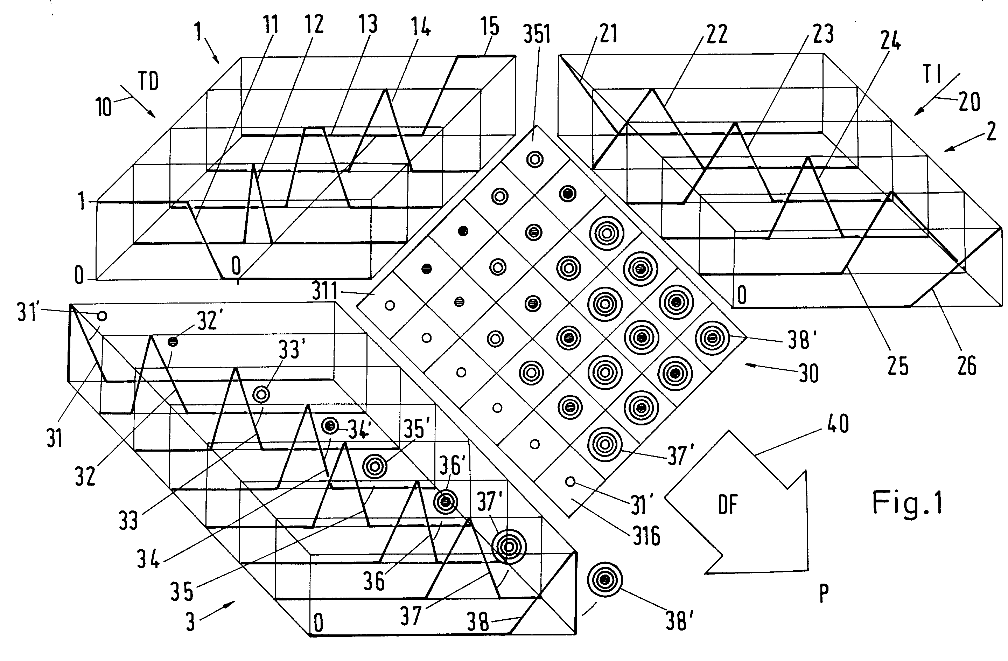

Schliesslich wird die vorgeschlagene Fuzzy-Regelung anhand der Zeichnungen erläutert. Es zeigt
- Fig. 1: eine graphische Darstellung zu der "Fuzzy-Logik"-Regelung des erfindungsgemässen Ablationsgeräts.

In dem Beispiel gemäss Fig.1 werden mit einem "Fuzzy-Logik"-Regler zwei Eingangsvariablen TD und TI in eine Ausgangsvariable P umgesetzt. P ist die Steuergrösse für die HF-Abgabeleistung, TD ist die Soll/Ist-Differenz zwischen Temperatur-Istwert und Solltemperatur, und TI ist der I-Anteil, wie er für den oben genannten PID-Regler bestimmt wird.

Das Verhalten des "Fuzzy-Logik"-Reglers ist durch eine Reihe von Regeln bestimmt, die hinsichtlich des zu lösenden Regelungsproblems entworfen worden sind. In einem ersten Schritt werden den Eingangsvariablen mittels einer "Fuzzifikation" "linguistische Variablen" zugeordnet, deren Werte "Terme" sind. Die Terme sind als unscharfe Mengen in Form von "Zugehörigkeitsfunktionen" definiert. Regeln, nämlich "wenn-dann"-Regeln, verknüpfen mittels "approximativen Schliessens" (auch "Inferenz" genannt) die Terme der Eingangsvariablen mit Termen einer linguistischen Variable, die der Ausgangsvariable zugeordnet sind und für die schliesslich mittels einer "Defuzzifikation" ein scharfer Wert der Ausgangsvariable berechnet wird.

Im Beispiel der Fig.1 sind - durch den Pfeil 10 symbolisiert - der Eingangsvariable TD fünf Terme mit Zugehörigkeitsfunktionen 11, ... 15 zugeordnet. In der gewählten Darstellung sind diese Funktionen 11, ... 15 auf Rechtecken eingezeichnet, die in einem Quader 1 auf parallelen Ebenen angeordnet sind. Die Grundlinie der Rechtecke stellen den Grundbereich der Stellgrösse dar, hier die Soll/Ist-Temperaturdifferenz (Nullpunkt in der Mitte). Zu einem gegebenen Wert der Stellgrösse lässt sich bezüglich den Funktionen 11, ... 15 jeweils ein bestimmter Zugehörigkeitsgrad bestimmen, der im Intervall zwischen 0 und 1 liegt.

Der zweiten Eingangsvariable TI sind entsprechend (Pfeil 20, Quader 2) sechs Zugehörigkeitsfunktionen 21, ... 26 zugeordnet. Acht Terme der Ausgangsvariable P sind durch Zugehörigkeitsfunktionen 31, ... 38 quantifiziert, und es sind diesen Termen zusätzlich Symbole 31', ... 38' zugeordnet. (Statt solcher Symbole werden normalerweise Wörter wie "niedrig", "mittel", "hoch" oder auch Modifikationen wie "sehr hoch" verwendet.)

Im Zentrum der graphischen Darstellung befindet sich eine Matrix 30 mit 5 Zeilen (mit der ersten Zeile bei der Eckzelle 311 und der letzten bei der Eckzelle 351) und 6 Spalten (mit der ersten Spalte bei der Eckzelle 311 und der letzten bei der Eckzelle 316). Diese Matrix 30 heisst "Regelblock" und zeigt, wie jeweils ein Paar von Termen der Eingangsvariablen TD und TI mit einem Term der Ausgangsvariable P verknüpft sind. Jede Zelle der Matrix enthält ein Symbol 31', ... 38', das einer der Funktionen 31, ... 38 entspricht.

Bei dem approximativen Schliessen wird zu einer bestimmten Eingangswertekombination mittels des Regelblocks eine Ausgangswertekombination bestimmt. Aus der resultierenden Kombination und in Abhängigkeit von den Zugehörigkeitsgraden der Stellgrössen wird bei der Defuzzifikation DF (Pfeil 40) die einzustellende Grösse P berechnet. Für diese Berechnung sind verschiedene Methoden bekannt, beispielsweise eine "Max-Min-Inferenz-Methode" (siehe Hans-Heinrich Bothe, "Fuzzy Logic", Springer-Verlag, 1993).

Die Regelung der Ablation kann erfindungsgemäss auch mit einer einzigen Eingangsvariablen durchgeführt werden. Diese Variable muss die Soll/Ist-Temperaturdifferenz TD sein. Mit Vorteil sind der zugehörigen linguistischen Variable mindestens drei Terme zugeordnet. Dabei sind die Terme als unscharfe Mengen auf einer Temperaturskala, d.h. einer Temperaturdifferenzen darstellenden Skala, definiert, wobei diese Skala einen negativen sowie einen positiven Bereich umfasst. Für eine Mehrzahl der Terme soll jeweils höchstens ein kleinerer Abschnitt, insbesondere ein Übergangsbereich, über dem negativen Bereich der Temperaturskala liegen. Dieses Erfordernis hängt mit dem besonderen Regelungsproblem bei der Ablation zusammen: Damit keine Koagulation an dem energieabgebenden Pol entsteht, soll bei Überschreiten der Solltemperatur (d.h. die Stellgrösse befindet sich im negativen Bereich der Temperaturskala) die HF-Abgabe auf ein Minimum reduziert werden. Für eine feine Abstufung der HF-Abgabe sind daher nur Terme hilfreich, die im positiven Bereich der Temperaturskala oder in unmittelbarer Umgebung des Nullpunkts Beiträge zur Regelung liefern. Bei dem Beispiel der Fig.1 sind es vier Terme dieses Typs (Zugehörigkeitsfunktionen 12, ... 15) und ein Term (Zugehörigkeitsfunktion 11), der mit der Abgabe einer minimalen Leistung verknüpft ist.

Bei der Definition der Terme für die Ausgangsvariable P wird dafür gesorgt, dass die feinste Einteilung im Hauptarbeitsbereich der HF-Quelle liegt.

Bei der Konzipierung des Regelblocks 30 wird darauf abgezielt, dass keine Koagulation entsteht. Zusätzlich wird mit den Regeln angestrebt, dass die für die Ablation benötigte Temperatur möglichst schnell erreicht wird, wobei aber gleichzeitig eine Überhitzung aufgrund eines Überschwingens unterbleiben soll.

Als weitere Eingangsvariablen der "Fuzzy-Logik"-Regelung kommen eine oder mehrere der folgenden Grössen in Frage:
- Ableitung des Temperatur-Istwerts nach der Zeit,
- Integral der Soll/Ist-Temperaturdifferenz über die Zeit (TI wie im Beispiel der Fig.1),
- abgelaufene Zeit seit Beginn der Ablation,
- einstellbarer Sollwert der Gewebetemperatur,
- Wert der Leistungsabgabe, der an den Eingang der Regelung rückgeführt wird.

## Patentansprüche

1. Ablationsgerät für intrakardiale Herzbehandlungen mit einer HF-Quelle, für deren Leistungsabgabe eine Temperaturregelung vorgesehen ist, und mit mindestens einem Ablationskatheter, der mindestens einen energieabgebenden Pol umfasst, wobei der oder jeder Pol mindestens einen Temperatursensor zur Messung einer Gewebetemperatur, nämlich eines Temperatur-Istwerts, enthält und mit dieser Messung jeweils die Soll/Ist-Differenz zwischen dem Temperatur-Istwert und einer vorgegebenen Solltemperatur bestimmbar ist,
dadurch gekennzeichnet, dass für die Beobachtung des bei der Ablation entstehenden thermischen Einflusses mehr als ein Temperatursensor vorgesehen ist und dass die Regelung Mittel umfasst, aufgrund derer die Leistungsabgabe so steuerbar ist, dass die grösste der durch die verschiedenen Temperatursensoren gemessenen Temperaturen einen vorgegebenen Grenzwert nicht wesentlich überschreitet.

2. Ablationsgerät nach Anspruch 1, dadurch gekennzeichnet, dass die Regelung der HF-Leistungsabgabe nur hinsichtlich der grössten gemessenen Temperatur vorgesehen ist.

3. Ablationsgerät nach Anspruch 1 oder 2, dadurch gekennzeichnet, dass als zusätzlicher Steuerwert für die HF-Leistungsabgabe eine Differenz zwischen den durch die verschiedenen Temperatursensoren gemessenen Temperaturen vorgesehen ist oder eine Mehrzahl von solchen Temperaturdifferenzen als Steuerwerte vorgesehen sind.

4. Ablationsgerät für intrakardiale Herzbehandlungen mit einer HF-Quelle, für deren Leistungsabgabe eine Temperaturregelung vorgesehen ist, und mit mindestens einem Ablationskatheter, der mindestens einen energieabgebenden Pol umfasst, wobei der oder jeder Pol mindestens einen Temperatursensor zur Messung einer Gewebetemperatur, nämlich eines Temperatur-Istwerts, enthält und mit dieser Messung jeweils die Soll/Ist-Differenz zwischen dem Temperatur-Istwert und einer vorgegebenen Solltemperatur bestimmbar ist,
dadurch gekennzeichnet, dass die Leistungsabgabe mittels einer "Fuzzy-Logik"-Regelung einstellbar ist, wobei mindestens eine der genannten Soll/Ist-Temperaturdifferenzen als Eingangsvariable der Regelung verwendet wird.

5. Ablationsgerät nach Anspruch 4, dadurch gekennzeichnet, dass der Soll/Ist-Temperaturdifferenz eine linguistische Variable mit mindestens drei Termen zugeordnet ist, wobei die Terme als unscharfe Mengen auf einer Temperaturskala mit einem negativen sowie einem positiven Bereich definiert sind, und dass für eine Mehrzahl der Terme jeweils höchstens ein kleinerer Abschnitt, insbesondere ein Übergangsbereich über dem negativen Bereich der Temperaturskala liegt.

6. Ablationsgerät nach Anspruch 4 oder 5, dadurch gekennzeichnet, dass als weitere Eingangsvariablen der "Fuzzy-Logik"-Regelung eine oder mehrere der folgenden Grössen vorgesehen sind:
- Ableitung des Temperatur-Istwerts nach der Zeit,
- Integral der Soll/Ist-Temperaturdifferenz über die Zeit,
- abgelaufene Zeit seit Beginn der Ablation,
- einstellbarer Sollwert der Gewebetemperatur,
- Wert der Leistungsabgabe, der an den Eingang der Regelung rückgeführt wird.

7. Ablationsgerät nach Anspruch 1 und Anspruch 4.

8. Ablationsgerät nach einem der Ansprüche 1 bis 7, dadurch gekennzeichnet, dass verschiedene Kathetertypen an die HF-Quelle anschliessbar sind, wobei die Temperatursensoren der Pole entweder Thermistoren oder Thermoelemente sind, und dass eine Sensortyp-Erkennung und entsprechende Mittel vorgesehen sind, aufgrund derer die Regelung für die Leistungsabgabe automatisch derart einschaltbar ist, dass die Durchführung der Leistungsregelung dem aktuellen Sensortyp entspricht.

9. Ablationsgerät nach Anspruch 8, dadurch gekennzeichnet, dass zusätzlich Mittel vorgesehen sind, mit denen mindestens ein Test durchführbar ist, der darauf gerichtet ist, ob angeschlossene Sensoren sinnvolle Temperaturwerte liefern, nämlich solche, die in zu erwartenden Bereichen liegen, und/oder ob hinsichtlich Kathetern mit Thermoelementen Vergleichsstellen der Thermoelemente sinnvolle Temperaturwerte liefern.

10. Ablationsgerät nach Anspruch 8 oder 9, dadurch gekennzeichnet, dass zusätzlich Mittel vorgesehen sind, mit denen die Messschaltung für die Temperaturmessung kalibrierbar und testbar sind.

11. Ablationsgerät nach einem der Ansprüche 1 bis 10, dadurch gekennzeichnet, dass Mittel vorgesehen sind, mit denen eine Überwachung einer Startphase durchführbar ist, wobei die Überwachung zur Verhinderung eines versehentlichen Starts in einem leistungsgeregelten Modus dienen soll.
